# EUROPEAN PATENT APPLICATION

(11) **EP 4 417 688 A1**
(43) Date of publication of application: **21.08.2024**
(21) Application number: 24156070.5
(22) Date of filing: 06.02.2024
(51) Int. Cl.: C12N 7/00

(54) **BACTERIOPHAGE COMPOSITION AGAINST FIREBLIGHT**

(30) Priority: 15.02.2023 BE 202305109
(71) Applicant: De Ceuster Meststoffen NV, 2280 Grobbendonk (BE)
(72) Inventor: Hanssen, Inge Renée Maria, 2280 Grobbendonk (BE); Vos, Christine, 2280 Grobbendonk (BE); Luypaert, Gil Karel, 2280 Grobbendonk (BE)
(74) Representative: Arnold & Siedsma

(57) **Abstract**

The present invention relates to a bacteriophage composition for the treatment a plant of the *Rosaceae* family to protect the plants from infection caused by *Erwinia* spp., and more specifically *Erwinia amylovora,* the causal agent of the disease called fireblight. The present invention further relates to methods for the treatment of a plant of the *Rosaceae* family to control *Erwinia* spp. infection using said composition and use of the bacteriophage composition or bacteriophage for the treatment of a plant of the *Rosaceae* family against *Erwinia* spp.

## Description

The present invention relates to a bacteriophage composition for the treatment of a plant of the *Rosaceae* family to protect the plants from infection caused by *Erwinia* spp., and more specifically *Erwinia amylovora,* the causal agent of the disease called fireblight. The present invention further relates to methods for the treatment of a plant of the *Rosaceae* family to control *Erwinia* spp. infection using said composition and use of the bacteriophage composition or bacteriophage for the treatment of a plant of the *Rosaceae* family against *Erwinia* spp.

Fireblight is a bacterial infectious disease affecting about 200 plant species of the family *Rosaceae* including apple- and pear orchards. Pears are the most susceptible, but apples, loquat, crab-apples, quinces, hawthorn, cotoneaster, Pyracantha, raspberry, blackberry, plum, and some other rosaceous plants are also susceptible. Fireblight is caused by the gram-negative bacterium *Erwinia amylovora* and is a serious concern to apple and pear producers in Europe, US, Japan and Australia, where fireblight can destroy an entire orchard in a single growing season.

Tissues affected by the symptoms of *Erwinia amylovora* include blossoms, fruits, shoots, branches and in an advanced stage of disease development even roots of the infected plants. A typical damage pattern of fireblight infections consists of flowers, blossoms and flower clusters of infected trees that appear water-soaked, then droop and shrivel, turning brown or black and remain attached through the growing season. The bark of affected trees at the base of blighted twigs becomes water soaked, then dark, sunken and dry. Young twigs and branches die from the terminal end and appear burned or deep rust colored and dead leaves and fruit remain on the branches. The pathogen spreads through the tree from the point of infection via the plant's vascular system, eventually reaching the roots and/or graft junction of the plant. Other than through the flowers, the bacterium can enter the plant through the stomata. Also highly susceptible to infection are lesions such as punctures caused by plant-sucking insects or damages to the orchards due to human interaction or climate conditions such as hail storms.

There is no known antimicrobial or effective treatment against fireblight infection. Antibiotics are known to be effective but are not allowed to be used in the EU due to strict legislation. In an attempt to prevent new infections, plants have been sprayed with bactericides including copper compounds (copper sulphide, copper (hydr)oxide, and copper oxychloride) but such control strategies are not always effective, and are definitely not sustainable, nor safe for consumers and workers and not environmentally friendly. The use of plant protection products is restricted to increasing environmental legislation, whereas the use of antibiotics such as streptomycin can lead to antibiotic resistance and is thus not allowed. Other control options include selecting for resistant cultivars, however most commercially successful cultivars at present still lack effective fireblight resistance. At present, when fireblight infections are observed, the only effective treatment for plants already infected is to prune off the affected branches and remove them from the area. Also further spread of the disease needs to be contained by measures such as pruning of tainted stems or branches. In extreme fireblight infections, the whole orchard needs to be removed and disposed of.

Considering the above, there is a need in the art for an effective, safe and sustainable treatment against fireblight infection in plants. In addition, there is a need in the art for a method for an effective and sustainable treatment of plants that are affected by fireblight. The treatment should provide a greener alternative and preferably not affected by the increasing limiting environmental legislation. Furthermore, the treatment should prevent or reduce the development of disease resistance against the treatment such as observed with the use of antibiotics for the treatment of fireblight in the US.

It is an object of the present invention, amongst other objects, to address the above need in the art. The object of present invention, amongst other objects, is met by the present invention as outlined in the appended claims.

Specifically, the above object, amongst other objects, is met, according to a first aspect, by the present invention by Bacteriophage composition for the treatment of a plant of the *Rosaceae* family against *Erwinia* spp. infection, wherein the composition comprises at least one bacteriophage, wherein said at least one bacteriophage is one or more selected from the group consisting of BAEA1, BAEA2, BAEA3 and BAEA4,
wherein BAEA1 is comprised of a genome sequence having at least 90% sequence identity, preferably 91%, 92%, 93%, 94%, 95%, 96%, 97% sequence identity, more preferably at least 98% sequence identity, even more preferably 100% sequence identity with SEQ ID No. 1,
wherein BAEA2 is comprised of the genome sequence having at least 90% sequence identity, preferably 91%, 92%, 93%, 94%, 95%, 96%, 97% sequence identity, more preferably at least 98% sequence identity, even more preferably 100% sequence identity with SEQ ID No. 2,
wherein BAEA3 is comprised of a genome sequence having at least 90% sequence identity, preferably 91%, 92%, 93%, 94%, 95%, 96%, 97% sequence identity, more preferably at least 98% sequence identity, even more preferably 100% sequence identity with SEQ ID No. 3, and, wherein BAEA4 is comprised of a genome sequence having at least 90% sequence identity, preferably 91%, 92%, 93%, 94%, 95%, 96%, 97% sequence identity, more preferably at least 98% sequence identity, even more preferably 100% sequence identity with SEQ ID No. 4.

BAEA1, BAEA2 and BAEA3 have a double stranded DNA genome having a length of about 261 to 262 kbp, represented by respectively SEQ ID No.1, SEQ ID No.2, and SEQ ID No.3. BAEA4 has a double stranded DNA genome having a length of about 75 kbp, represented by SEQ ID No.4.

Bacteriophages are a group of viruses that rely on bacteria for survival and replication. Bacteriophages consist of genetic material (DNA or RNA, single- or double stranded, circular or linear; mostly dsDNA is found) surrounded by a protein capsid as is the case for the bacteriophages comprised in the composition of present invention. Bacteriophages are the most abundant biological entity in the biosphere with an estimated number of 10³¹ individuals, which is at least ten times higher than the number of bacterial cells. They can be found in saltwater, freshwater, soil, plants and animals as well as in the human digestive and genitourinary tracts and even on the skin. Bacteriophages are the natural enemies of bacteria and fit nicely within the scope of the implemented integrated pest management strategies (IPMs) as a standard for crop protection (e.g. Directive 2009/128/EC). Genome sequencing has revealed a huge diversity in bacteriophage genomes, at current over 19.000 genomes of viruses are included in the NCBI database, the majority of which being bacteriophages.

According to a preferred embodiment, the present invention relates to the bacteriophage composition, wherein said at least one bacteriophage is at least two, preferably at least three bacteriophages selected from the group consisting of BAEA1, BAEA2, BAEA3 and BAEA4, preferably at least BAEA1, BAEA2 and BAEA3, more preferably all four bacteriophages.

According to yet another preferred embodiment, the present invention relates to the bacteriophage composition, wherein the *Erwinia* spp. infection is caused by the *Erwinia amylovora* (*E. amylovora*) bacterium. For example the *Erwinia amylovora* represented by isolate DSM 34444 as deposited at Leibniz-Institut DSMZ-Deutsche Sammlung von Mikroorganismen und Zellkulturen GmbH, Inhoffenstr. 7B, D38124 Braunschweig, Germany on 23 November 2022. Bacteriophage host recognition occurs via receptors on the cell surface. Bacteriophages have a narrow host range and are generally considered species- or even strain-specific. The host specificity of bacteriophages can vary per genus. Some bacteriophages can infect members of multiple bacterial genera while some are specific for particular isolates or groups of isolates of closely related host species. In order to successfully infect a host bacterium, binding of the bacteriophage to the bacterium is needed with specialized surface receptors. As a consequence, it is unlikely that bacteriophages that are able to infect bacteria with specific receptors are also able to infect commensal bacteria. Experiments show that the bacteriophages of the composition of present invention are strictly specific to *Erwinia* spp., preferably including *Erwinia amylovora, Erwinia pyrifoliae, Erwinia piriflorinigrans* and *Erwinia uzenensis.* Therefore, the bacteriophage composition of present invention does also not affect other bacteria that are beneficial to the plant or orchard on which the composition is applied for treatment of fireblight.

According to another preferred embodiment, the present invention relates to the bacteriophage composition, wherein the plant of the *Rosaceae* family is selected from the group consisting of apple, pear, raspberry, blueberry, blackberry, crapapple, loquat, strawberry, apricot, plum, almond, rose, hawthorn and quince, preferably apple or pear.

According to another preferred embodiment, the present invention relates to the bacteriophage composition, wherein BAEA1 is obtainable from the deposit DSM 34445, wherein BAEA2 is obtainable from the deposit DSM 34446, wherein BAEA3 is obtainable from the deposit DSM 34447, and wherein BAEA4 is obtainable from the deposit DSM 34448. The above specified bacteriophages were all deposited at Leibniz-Institut DSMZ-Deutsche Sammlung von Mikroorganismen und Zellkulturen GmbH, Inhoffenstr. 7B, D38124 Braunschweig, Germany on 23 November 2022.

According to another preferred embodiment, the present invention relates to the bacteriophage composition, wherein the composition is a suspension concentrate, an aqueous solution, granule or powder, preferably suspension concentrate.

The composition of present invention may further comprise additives or adjuvants for improved efficacy, such as UV protectors for improved stability of the bacteriophage composition under sunlight, spreaders for increased contact surface and better uptake, or rain-fastness enhancers for improved persistence in rainy weather. Inclusion of spreaders in the composition of present invention provides for an improved spread of the bacteriophages when applied due to a larger contact surface, resulting in improved take up and improved treatment of fireblight. Rain-fastness enhancers for example prevent washout of the bacteriophages during rainfall, preventing a reduced efficacy and maintaining high MOIs (Multiplicity of Infection). The MOI or multiplicity of infection refers to the ratio of bacteriophages to target bacterial cells and is an important value in bacteriophage based control of bacterial diseases. The composition of present invention may further include sticker, emulsifier, wetting agent, buffering agent, anti-foam agent, drift control agent, additive for increased surface contact, reduced runoff and increased leaf penetration in various weather conditions.

According to yet another preferred embodiment, the present invention relates to the bacteriophage composition, wherein the composition comprises 1×10⁷ to 1×10¹² PFU/mL of bacteriophages, preferably 1×10⁸ to 1×10¹¹ PFU/mL, more preferably 1×10⁹ to 1×10¹⁰ PFU/mL. Experiments show that a concentration of 5×10⁹⁻¹⁰ PFU/mL is especially suitable against the *Erwinia* spp. infection in apple and pear.

The present Invention, according to a second aspect, relates to a method for the treatment of a plant of the *Rosaceae* family against *Erwinia* spp. infection, wherein a bacteriophage composition of present invention is applied to the plant, fruit, blossom, shoot, trunk, root, or other plant parts.

According to a preferred embodiment, the present invention relates to the method wherein the method is a preventive and/or curative method. The bacteriophage composition of present invention can be used as preventive treatment to prevent the plant from being infected by *Erwinia* spp. and affected by fireblight. Furthermore, and as shown in the experiments, when infected by *Erwinia* spp. plants can be effectively treated by application of the composition of present invention to said plants.

According to another preferred embodiment, the present invention relates to the method wherein the application of the bacteriophage composition to the plant is performed by spraying, watering of the plant, irrigation, soil/substrate drenching, via a drip irrigation system on the soil/substrate or solid application via granules, dusting, dipping, trunk injection, via insect transmission, preferably by spraying.

According to another preferred embodiment, the present invention relates to the method, wherein the bacteriophage composition is applied at a dosage in the range of 0,375 to 6 L/ha LWA, preferably 0,75 to 4,5 L/ha LWA, more preferably 1,5 to 3 L/ha LWA. Preferably the bacteriophage composition is applied at a dosage of at least 0,75 L/ha LWA, preferably at least 1,5L/ha LWA, more preferably at least 3 L/ha LWA, even more preferably 6 L/ha LWA. The combination of concentration of the product (number of viable bacteriophages per ml of product, see above when discussing the MOI), and the dosage applied on the plant (in L per ground surface or ha leaf wall area (LWA)) of the product are crucial to obtain an optimal efficacy. Ha LWA is a dose expression for plant protection products referring to an amount (i.e. L) per treated hectare (ha) leaf wall area (LWA) of the plant, as further described in (Bulletin OEPP/EPPO Bulletin (2012) 42 (3), 409-41). Experiments show that when the bacteriophage composition is applied to the leaves of a plant suffering from fireblight the application at a dose of at least 0,75 L/ha LWA is already sufficient for reducing the infection pressure. As indicated in the experiments, preferably 3 L/ha LWA is used for the treatment of fireblight in an apple or pear orchard.

According to another preferred embodiment, the present invention relates to the method, wherein the bacteriophage composition is applied to the plant at an application frequency selected from the group consisting of once a year, once a month, twice a month, four times a month, every two weeks, once a week, twice a week and daily, preferably once or twice a week.

The method of present invention is able to prevent or reduce fireblight in infected plants by killing the *Erwinia* spp., wherein bacterial infection or fireblight incidence is reduced by at least 25%, preferably at least 40%, more preferably at least 50% in comparison to untreated plants.

The present invention, according to a further aspect, relates to the use of a bacteriophage composition or bacteriophage as described or claimed herein for the treatment or control of a plant of the *Rosaceae* family affected by fireblight infection. Isolated bacteriophage BAEA1 (SEQ ID No. 1), BAEA2 (SEQ ID No. 2), BAEA3 (SEQ ID No. 3) and/or BAEA4 (SEQ ID No. 4) can be used alone or in combination for the treatment of a plant of the *Rosaceae* family affected by fireblight infection.

The present invention, according to a further aspect, relates to an isolated bacteriophage species for the treatment of a plant of the *Rosaceae* family against *Erwinia* spp., comprised of a genome sequence having at least 95% sequence identity with SEQ ID No. 1 and/or SEQ ID No.2, preferably wherein said bacteriophage species is comprised of a bacteriophage BAEA1 or BAEA2, comprising a genome sequence of SEQ ID No. 1 or SEQ ID No. 2, respectively. A phage is considered to belong to the same species if the identity at the genome level is more than 95% and if there is a clear genome synteny.

The present invention, according to a further aspect, relates to an isolated bacteriophage species for the treatment of a plant of the *Rosaceae* family against *Erwinia* spp., comprised of a genome sequence having at least 95% sequence identity with SEQ ID No. 3, preferably wherein said bacteriophage species is comprised of a bacteriophage BAEA3 comprising a genome sequence of SEQ ID No. 3.

The present invention, according to a further aspect, relates to an isolated bacteriophage for the treatment of a plant of the *Rosaceae* family against *Erwinia* spp., comprised of a genome sequence having at least 98% sequence identity with SEQ ID No. 4, preferably wherein said bacteriophage is comprised of a bacteriophage BAEA4 comprising a genome sequence of SEQ ID No. 4.

The present invention will be further detailed in the following examples and figures wherein:

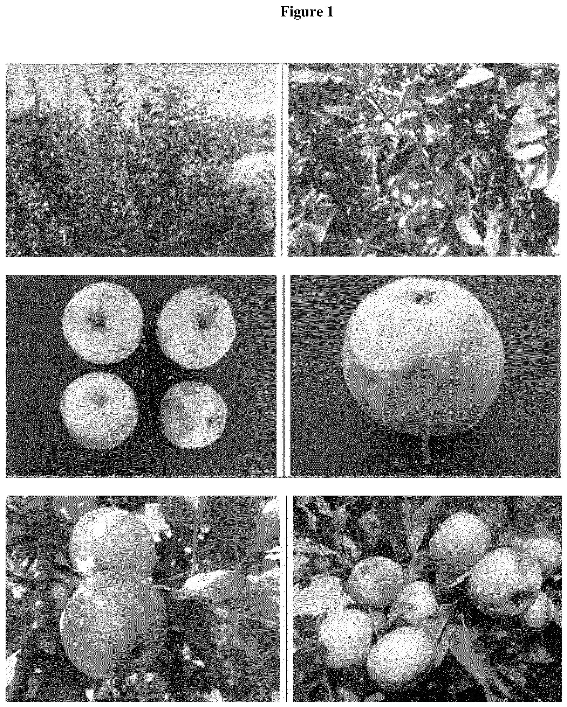

- **Figure 1:**: Shows apple trees and apples (Topaz/M9 variety) that have been infected by *Erwinia amylovora* suffering from fireblight. The upper panels show the apple orchard not treated with the composition of present invention and affected by fireblight. The middle panels show the fruits of these trees that did not receive treatment with the composition of present invention. The lower panels show part of the trees and apple that has been treated by the bacteriophage composition of present invention at two different dosage rates (left panel is 1,5 L/ha LWA, right panel is 3 L/ha LWA). The fireblight was effectively controlled by the bacteriophage composition of present invention.

### Examples

### Example 1 - sequencing of BAEA1, BAEA2, BAEA3 and BAEA4 bacteriophage

The full genomes of the *Erwinia amylovora* bacteriophages comprised in the composition of present invention were sequenced using Illumina sequencing. From each bacteriophage total bacteriophage genomic DNA was extracted as previously described in (Kot, W. Genome sequencing of dsDNA-containing bacteriophages directly from a single plaque. Methods Mol Biol 1681, 179-184 (2018). Briefly, a 90 µl aliquot of the provided bacteriophage stock was mixed with 10 µl Dnase Ibuffer (ThermoFisher) and filtered using a 0,45 µm ultrafiltration spin-column (Merck Millipore) to remove any possibly remaining cell debris. Subsequently, the mixture was treated with 5 U Dnase I (30', 37°C) (ThermoFisher) to get rid of all external host DNA. After addition of 10 µl 50 mM EDTA and 10 µl 1% SDS, the bacteriophage capsids were digested with 3 U proteinase K (45', 55°C) (ThermoFisher). Finally, the total bacteriophage genomic DNA was purified using the DNA Clean & Concentrator-5 kit (Zymo Research). The eluted bacteriophage DNA was directly used for Illumina sequencing (Illumina MiniSeq platform). Using MEGA X v 10.1.89, bacteriophage genomes were then aligned to the closest type species as identified by BLASTn10 and/or Viptree v1.911.

To study the taxonomy of the viruses in more detail, the virus intergenomic distance between the most related phages (as identified by BLASTn and Viptree) and the three *E*. *amylovora* phages was calculated, showing that BAEA1 and BAEA2 are belonging to the same new species (i.e. they share >95% nucleotide similarity), while BAEA3 forms another new species according to the International Committee for the Taxonomy of Viruses (ICTV) guidelines. Bacteriophages BAEA1, BAEA2 and BAEA3, (SEQ ID No.1, SEQ ID No.2, SEQ ID No.3, respectively) have a genome length of about 261-262 kbp. The closest hits are bacteriophage Alexandra (70 - 89% coverage, 80,31 - 87,98% identity) and Dickeya bacteriophage AD1 (71 - 86% coverage, 79,95 - 87,29% identity), belonging to the *Alexandravirus* genus. This genus is currently not yet classified to a particular family of the Caudoviricetes class. As Alexandra and AD1 are currently classified both within the same *Alexandravirus* genus, we can argue that BAEA1, BAEA2 and BAEA3 may also belong to the *Alexandravirus* genus. However, they do not belong to previously described species, as their DNA sequence identity to other previously described bacteriophages is lower than 95%, this percentage forming the cut-off value for sequence identity within one viral species (ICTV).

Similar as described above the full genome of bacteriophage BAEA4 was sequenced and characterized. BAEA4 did not relate to the above two new bacteriophage species and was therefore identified as another species. BAEA4 has a genome length of 75 kbp (SEQ ID No.4). BAEA4 may be classified in a species of a genus of the Schitoviridae family, characterized by a virion-associated RNA polymerase.

Furthermore, after annotation of the genomes, no indications were found that would suggest these four bacteriophages have a temperate lifestyle. It was concluded that the identified bacteriophages are strictly lytic, based on their known encoded proteins (genbank files) and on their related bacteriophages (BLASTn and Viptree analysis).

### Example 2 - Host specificity of BAEA1, BAEA2, BAEA3 and BAEA4 bacteriophages

The Host-Specificity method was performed in accordance with the Good Laboratory Practice Standards (EPA 40 CFR § 160) and is a test to confirm that the bacteriophages in the products are specific to the problematic bacteria that they were isolated to destroy, and not infective to other closely related organisms or bacterial flora within plants, soil, or humans. Bacteriophages *BAEA1, BAEA2, BAEA3,* and preferably *BAEA4* that are present in the composition of present invention were tested for their host specificity on different bacterial species including *Pantoea agglomerans* (ATCC 27155), *Azotobacter chroococcum* (ATCC 9043), *Ensifer meliloti* (ATCC 9930), *Pseudomonas aeruginosa* (ATTC 15442), *Klebsiella pneumoniae* (ATCC 13883), *Enterobacter aerogenes* (ATCC 13048), *Bacillus subtilis* (ATCC 6051), *Streptomyces griseus* (ATCC 23345), *Staphylococcus epidermidis* (ATCC 14990), *Escherichia coli* (ATCC 11775), *Proteus vulgaris* (ATCC 12454), *Enterecoccus faecalis* (ATCC 19433), *Salmonella enterica serovar enteritidis* (ATCC 4931), *Citrobacter freundii* (ATCC 8090), and *Serratia marcescens* (ATCC 13880). The test substances are bacteriophage concentrates. For storage, they are refrigerated at 4°C.

Each bacteriophage was tested against fifteen organisms and observed for formation of lysis. A simple spotting assay is used, which is a quick method to determine lysis. Briefly, bacteria were inoculated on LBA plates and incubated overnight at 30°C. Next, 10 µl of each bacteriophage was spotted on the plate comprising the bacteria and subsequently incubated for 18 to 22 hours at 30°C. Next, plates were examined by eye for lysis or plaques of the bacteria for each bacteriophage spot. The lack of lysis or plaques indicates that the bacteriophages are not active against the tested host.

No cell lysis or cell plaques were observed when the indicated bacteria were exposed to the selected bacteriophages, whereas *Erwinia amylovora* bacteria (e.g. the *Erwinia amylovora* represented by deposit DSM 34444 as deposited at Leibniz-Institut DSMZ-Deutsche Sammlung von Mikroorganismen und Zellkulturen GmbH, Inhoffenstr. 7B, D38124 Braunschweig, Germany on 23 November 2022) did show cell lysis and cell plaques when exposed to the bacteriophages. It can be concluded that the selected bacteriophages are host specific for the *Erwinia amylovora* bacteria.

### Example 3 - Host range of BAEA1, BAEA2, BAEA3 and BAEA4 bacteriophages on Erwinia amylovora isolates

The host range of the BAEA1, BAEA2, BAEA3 and BAEA4 bacteriophages on *Erwinia amylovora (E. amylovora)* isolates was investigated using an EOP assay (= efficiency of plating) on 71 different isolates of *E. amylovora,* that originated from all over the world including EU (DE, NL, IT, SP, BE, PL, AU), US, CA and Morocco. This is a relative test to measure the activity of the bacteriophages against the bacterial isolates.

Briefly, the bacteriophage titer was estimated by the soft agar overlay method (Kropinski et al., 2009). Briefly, soft agar (dipeptone 5 g/L, yeast 2 g/L, MgSO₄.7H₂O 2 g/L, K₂HPO₄ 1 g/L, glycerol 5 mL/L, agar 4 g/L, pH 6,5±0,1) containing 100 µL of a bacterial cell suspension at optical density (OD₆₀₀nm) 0,5 and 100 µL of bacteriophages at 1E+03, 1E+02, 1E+01 plaques-forming unit per millilitre (PFU/mL) was poured over bottom agar plates (peptone 5 g/L, yeast 2 g/L, MgSO₄.7H₂O 2 g/L, KH₂PO₄ 0,25 g/L, glycerol 5 mL/L, NaOH 0,01 g/L, agar 6 g/L, pH 6,4±0,1). Each experiment was carried out in triplicate. Results were visualized as zones of clearing (plaques) in the bacterial lawn. Clear plaques were counted after 72 h of incubation at 27°C and reported as PFU/mL. Host specificity was scored on the basis of the PFU/mL, wherein a value of <0,1 PFU/mL gives a score A, between 0,1 - 0,5 PFU/mL gives a score B, between 0,5 - 1 PFU/mL gives a score C, and >1 PFU/mL gives a score D, Table 1.

**Table 1. Scoring of host range of the bacteriophages on isolates of E. amylovora**

| **Score** | | | | |
|---|---|---|---|---|
| **Isolates** | **BAEA2** | **BAEA3** | **BAEA1** | **BAEA4** |
| **EA_1** | B | C | C | C |
| **EA_2** | D | C | C | C |
| **EA_5** | B | C | C | B |
| **EA_6** | B | C | C | A |
| **EA_10** | B | C | C | C |
| **EA_11** | C | C | D | B |
| **EA_12** | B | C | C | C |
| **EA_13** | B | C | C | C |
| **EA_14** | B | C | C | C |
| **EA_15** | C | D | D | C |
| **EA_16** | D | D | D | C |
| **EA_17** | D | D | D | C |
| **EA_19** | D | D | D | D |
| **EA_20** | D | D | D | C |
| **EA_21** | D | D | D | C |
| **EA_22** | C | D | D | C |
| **EA_23** | D | D | D | C |
| **EA_24** | D | D | D | C |
| **EA_25** | D | D | D | C |
| **EA_26** | D | D | D | C |
| **EA_28** | C | C | C | C |
| **EA_29** | C | B | C | C |
| **EA_30** | C | C | C | D |
| **EA_32** | C | C | C | D |
| **EA_33** | C | A | D | B |
| **EA_34** | C | C | D | B |
| **EA_35** | C | A | D | C |
| **EA_36** | B | C | C | B |
| **EA_37** | B | C | C | C |
| **EA_38** | C | D | D | A |
| **EA_39** | C | C | D | A |
| **EA_40** | B | C | C | C |
| **EA_41** | B | C | D | B |
| **EA_42** | B | D | D | A |
| **EA_43** | D | D | D | A |
| **EA_44** | B | D | D | A |
| **EA_45** | B | C | D | A |
| **EA_46** | B | C | C | C |
| **EA_47** | D | D | D | A |
| **EA_49** | A | A | A | C |
| **EA_50** | B | C | C | D |
| **EA_51** | C | D | D | B |
| **EA_55** | C | C | D | B |
| **EA_59** | C | C | D | C |
| **EA_63** | C | C | C | D |
| **EA_72** | D | C | D | D |
| **EA_74** | D | C | C | D |
| **EA_75** | C | C | D | C |
| **EA_78** | C | C | D | D |
| **EA_81** | C | D | D | A |
| **EA_82** | C | D | D | A |
| **EA_84** | C | B | D | B |
| **EA_85** | C | A | A | B |
| **EA_86** | C | B | D | B |
| **EA_87** | C | B | D | B |
| **EA_88** | C | B | D | B |
| **EA_89** | C | B | D | D |
| **EA_90** | C | C | D | D |
| **EA_91** | C | C | D | C |
| **EA_92** | C | C | D | C |
| **EA_93** | C | C | D | D |
| **EA_94** | C | C | D | D |
| **EA_95** | C | C | D | D |
| **EA_96** | C | C | D | D |
| **EA_97** | C | C | C | D |
| **EA_98** | C | C | D | D |
| **EA_99** | B | C | D | D |
| **EA_100** | C | C | C | C |
| **EA_101** | A | A | A | C |
| **EA_102** | C | C | C | B |
| **EA_103** | D | D | C | A |
| **EA_104** | C | C | C | C |

Isolates with scores of B, C or D are considered as susceptible. The above results show that the selected bacteriophages are very effective against *Erwinia* spp. and more specifically *Erwinia amylovora.* As can be seen from Table 1, most *Erwinia* isolates are susceptible to infection by all four bacteriophages, although the level of activity can vary where specific isolates are controlled more efficiently than others, depending on the bacteriophage-bacterial isolate combination. Furthermore, these results support the idea that a combination (cocktail) of two or more bacteriophages can control the disease even more efficiently in view of bacterial diversity of the *Erwinia amylovora* spp.

### Example 4 - Efficacy of bacteriophage composition comprising BAEA1, BAEA2, and BAEA3 against fireblight infection in apple

Apples (Goldrush and Topaz variety) were tested in the field (Romania, 2285 trees/ha) on the efficacy of the composition of present invention comprising the BAEA1, BAEA2, and BAEA3 bacteriophages. Apple trees were infected with *Erwinia amylovora* by natural infestation based on the prevalence of *E. amylovora* in the previous years. One group of trees (about 50%) was treated with the bacteriophage composition (5×10⁹ PFU/mL) by foliar spray and the other group did not receive the bacteriophage treatment but was treated by a known antimicrobial (Aliette 80 WP, Bayer), according to manufacturer's instructions. Treatment with the bacteriophage composition of present invention was furthermore performed in three different concentrations of application, 0,75 L/ha LWA, 1,5 L/ha LWA and 3 L/ha LWA. During the experimentation period, no phytotoxicity or other detrimental effects were noticed in the plants treated with the bacteriophage composition nor with standard product Aliette 80 WP. The experiment was conducted for 5 months from May to August 2021 in a 12-year-old apple experimental plot, organized and conducted with high quality fruits. The bacteriophage composition was applied during these months with a 5-6 day interval, just before sunset, using an STIHL 430SR atomizer.

Fireblight incidence, severity and damage rates on flowers, shoots, and fruits were evaluated after the first, second and third application until harvest, in middle of September. It was observed that the microclimate conditions led to a very quick fireblight propagation during May and June 2021 and that the most critical period for fireblight infection and disease development was in 2021 from early May till the end of July. Another time point with a high disease pressure was noticed in the first half of September. Applied at a dose 3,0 L/ha LWA, the bacteriophage composition of present invention was highly effective in control of the fireblight - *Erwinia amylovora* - Burill Winslow on apples varieties, on rosettes, growing shoots young and growing fruits (see Figure 1). Also, good results were obtained with the bacteriophage compositions at lower dosage 1,5 L/ha LWA. Even at lower dose of 0,75 L/ha LWA provided a decent protection of flower clusters, rosettes, shoots and fruits. Only 7,8% (Goldrush) and 11,3% (Topaz) of the apples were unaffected by fireblight in the group that did not receive the bacteriophage composition. In the treated group about 85% of the apples remained unaffected at 0,75 L/ha LWA, 93,5% at 1,5 L/ha LWA and 100% at 3 L/ha LWA, independent of the apple variety. Furthermore, the bacteriophage composition of present invention provided a very efficient control of damages to the trees, including their rosettes and shoots, even applied at the lowest dose 0,75 L/ha LWA. These results show that the bacteriophage composition of present invention provides an effective treatment for fireblight in apple.

### Example 5 - efficacy of bacteriophage composition comprising BAEA1 , BAEA2, and BAEA3 against fireblight infection in pear

Pear (Conference variety) was tested in the field (Scarnafigi - Piedmont Region, Northern Italy) on the efficacy of the composition of present invention comprising the BAEA1, BAEA2, and BAEA3 bacteriophages. The experiment was conducted from April to June 2022. Pear trees were infected with *Erwinia amylovora* by natural infestation based on the prevalence of *Erwinia amylovora* in the previous years. One group of trees (about 50%) was treated with the bacteriophage composition (5×10⁹ PFU/mL) by foliar spray and the other group did not receive the bacteriophage treatment but was treated by a known antimicrobial (SERENADE ASO, Bayer), according to manufacturer's instructions. Treatment with the bacteriophage composition of present invention was furthermore performed in different dosages of application, 0,375, 0,75, 1,5, 3 and 6 L/ha LWA. "L/ha LWA" is defined herein as Liters Product per 10.000 Square Meters (ha) Leaf Wall Area. During the experimentation period, no phytotoxicity or other detrimental effect were noticed in the plants treated with the bacteriophage composition nor with standard product SERENADE ASO. Products were applied four times during the flowering stages, on the 4th, 8th, 11th and 19th of April.

Fireblight incidence, severity and damage rates on flowers, shoots, and fruits were evaluated. During the trial period, assessments were performed on the number of exudates on trees during flowering, as well as one assessment on the percentage of infected flower clusters (200 flower clusters/plot) and three assessments on the percentage of infected shoots (200 shoots/plot) after the flowering, counting the number of injured clusters by *Erwinia amylovora.* Infected flower clusters and shoots were removed at each assessment from the trial site. In the presence of a severe level of infection by *Erwinia amylovora,* both the bacteriophage composition of present invention and the known antimicrobial as reference products provided a remarkable disease control compared to the untreated control.

On the 11th of April assessment on the number of exudates on trees was performed during the flowering. On the plot comprising the untreated trees on average 7,3 exudates/plot were recorded. On the treated plots significantly lower number of exudates was recorded, ranging between 3,8 and 5,0 exudates/plot. Efficacy results, calculated according Abbott's formula, on number of exudates ranged between 27,9 - 44,5%, without significant differences among the treatments.

After the flowering, another assessment was performed on the percentage of infected flower clusters, carried out on the 26th of April, when on the untreated control was recorded 13.8% of infected flower clusters. On the treated plots was recorded significantly lower incidence, with highest values on 0,375 L/ha LWA treatment (7,5%), followed by 0,75 L/ha LWA treatment (5,0%), while on other treatments were recorded significantly lower values, ranging between 1,0 - 3,5%. Hence, the efficacy results were lowest on 0,375 L/ha LWA treatment (40,6%), followed by 0,75 L/ha LWA treatment (61,2%), while on other treatments the efficacy ranged between 73,2% and 93,7%.

First assessment on shoots was carried out on the 17th of May, when on the untreated control was recorded 10,1% of infected shoots, while on the treated plots incidence ranged between 0,6 - 3,4%. Efficacy results on shoots ranged between 52,4% and 76,4%, without significant differences between the treatments. At the following assessment (May 30th), on the untreated control was recorded 11,0% of infected shoots, while on the treated plots incidence ranged between 5,3 - 10,6%. Efficacy results on this date were highest on SERENADE ASO (51,8%) and 6 L/ha LWA treatment (51,5%), followed by 3 L/ha LWA treatment (35,3%) and 1.5 L/ha LWA treatment (38,5%), while the lowest efficacy was recorded on 0,75 L/ha LWA treatment (22,6%) and 0,375 L/ha LWA treatment (10,1%). By the time of the final assessment (June 10th), on the untreated control was recorded 21,9% of infected shoots, while on the treated plots incidence ranged between 7,3 - 15,4%. Efficacy results on this date were again highest on SERENADE ASO (66,2%) and 6 L/ha LWA treatment (60,1%), followed from high to lower treatment in line with what was observed earlier (56,1%), (56,3%), (35,1%), and (26,6%).

The composition of present invention showed a clear dosage rate response in control of the fireblight - *Erwinia amylovora,* recording higher disease control when applied at higher dosage rates. Furthermore, when applied from a dosage rate of 3 L/ha LWA or higher, the test product achieved equivalent disease control as the reference product SERENADE ASO applied at 8 L/LWA. However, the dosage rate of 1,5 L/ha LWA also had a significant positive effect in the control of *Erwinia amylovora* when compared to the untreated control. No phytotoxic symptoms were observed on the crop in all the treatments where the different products were applied, thus showing the full selectivity on pear, Conference variety.

## Claims

1. Bacteriophage composition for the treatment of a plant of the *Rosaceae* family against *Erwinia* spp. infection, wherein the composition comprises at least one bacteriophage, wherein said at least one bacteriophage is one or more selected from the group consisting of BAEA1, BAEA2, BAEA3 and BAEA4,
wherein BAEA1 is comprised of a genome sequence having at least 90% sequence identity, preferably 98% sequence identity with SEQ ID No. 1,
wherein BAEA2 is comprised of the genome sequence having at least 90% sequence identity, preferably 98% sequence identity with SEQ ID No. 2,
wherein BAEA3 is comprised of a genome sequence having at least 90% sequence identity, preferably 98% sequence identity with SEQ ID No. 3, and,
wherein BAEA4 is comprised of a genome sequence having at least 90% sequence identity, preferably 98% sequence identity with SEQ ID No. 4.

2. Bacteriophage composition according to claim 1, wherein said at least one bacteriophage is at least two, preferably at least three bacteriophages selected from the group consisting of BAEA1, BAEA2, BAEA3 and BAEA4, preferably at least BAEA1, BAEA2 and BAEA3, more preferably all four bacteriophages, preferably wherein BAEA1 is obtainable from the deposit DSM 34445, wherein BAEA2 is obtainable from the deposit DSM 34446, wherein BAEA3 is obtainable from the deposit DSM 34447, and wherein BAEA4 is obtainable from the deposit DSM 34448.

3. Bacteriophage composition according to claim 1 or 2, wherein the *Erwinia* spp. infection is caused by the *Erwinia amylovora* bacterium.

4. Bacteriophage composition according to any one of the claims 1 to 3, wherein the plant of the *Rosaceae* family is selected from the group consisting of apple, pear, raspberry, blueberry, blackberry, crapapple, loquat, strawberry, apricot, plum, almond, rose, hawthorn, and quince, preferably apple or pear.

5. Bacteriophage composition according to any one of the claims 1 to 4, wherein the composition is a suspension concentrate, an aqueous solution, granule or powder, preferably suspension concentrate.

6. Bacteriophage composition according to any one of the claims 1 to 5, wherein the composition comprises 1×10⁷ to 1×10¹² PFU/mL of bacteriophages, preferably 1×10⁸ to 1×10¹¹ PFU/mL, more preferably 1×10⁹ to 1×10¹⁰ PFU/mL.

7. Bacteriophage composition according to any one of the claims 1 to 6, wherein the composition further comprises additives or adjuvants selected from the group consisting of a UV protector, spreader, rainfastness enhancer, sticker, emulsifier, wetting agent, buffering agent, anti-foam agent, drift control agent, additive for increased surface contact, reduced runoff and increased leaf penetration in various weather conditions.

8. A method for the treatment of a plant of the *Rosaceae* family against *Erwinia* spp. infection, wherein a bacteriophage composition of any one of the claims 1 to 7 is applied to the plant, fruit, blossom, shoot, trunk, root, or other plant parts, preferably wherein the application of the bacteriophage composition to the plant is performed by spraying, watering of the plant, irrigation, soil/substrate drenching, via a drip irrigation system on the soil/substrate or solid application via granules, dusting, dipping, trunk injection, via insect transmission, preferably by spraying, and/or preferably wherein the method is a preventive and/or curative method..

9. The method according to the claim 8 , wherein the bacteriophage composition applied at a dosage in the range of 0,375 to 6 L/ha LWA, preferably 0,75 to 4,5 L/ha LWA, more preferably 1,5 to 3 L/ha LWA, and/or preferably wherein the bacteriophage composition is applied to the plant at an application frequency selected from the group consisting of once a year, once a month, twice a month, four times a month, every two weeks, once a week, and twice a week, daily, preferably once a week..

10. Use of a bacteriophage composition of any one of the claims 1 to 7 for the treatment or control of *Erwinia* spp. infection of a plant of the *Rosaceae* family.

11. An isolated bacteriophage species for the treatment of a plant of the *Rosaceae* family against *Erwinia* spp., comprised of a genome sequence having at least 95% sequence identity with SEQ ID No. 1 and/or SEQ ID No.2, preferably wherein said bacteriophage species is comprised of a bacteriophage BAEA1 or BAEA2, comprising a genome sequence of SEQ ID No. 1 or SEQ ID No. 2, respectively.

12. An isolated bacteriophage species for the treatment of a plant of the *Rosaceae* family against *Erwinia* spp., comprised of a genome sequence having at least 95% sequence identity with SEQ ID No. 3.

13. Isolated bacteriophage species according to claim 12, wherein said bacteriophage species is comprised of a bacteriophage BAEA3 comprising a genome sequence of SEQ ID No. 3.

14. An isolated bacteriophage for the treatment of a plant of the *Rosaceae* family against *Erwinia* spp., comprised of a genome sequence having at least 98% sequence identity with SEQ ID No. 4.

15. Isolated bacteriophage according to claim 14, wherein said bacteriophage is comprised of a bacteriophage BAEA4 comprising a genome sequence of SEQ ID No. 4.
